Europäisches Patentamt

European Patent Office

Office européen des brevets

(19)

(11) Veröffentlichungsnummer: **0 542 290 A1**

(12) **EUROPÄISCHE PATENTANMELDUNG**

(21) Anmeldenummer: **92119444.5**

(22) Anmeldetag: **13.11.92**

(51) Int. Cl.5: **C07C 17/38**, C07C 19/08, C01B 7/19

(30) Priorität: **15.11.91 DE 4137600**

(43) Veröffentlichungstag der Anmeldung:
**19.05.93 Patentblatt 93/20**

(84) Benannte Vertragsstaaten:
**BE DE DK ES FR GB IT NL**

(71) Anmelder: **HOECHST AKTIENGESELLSCHAFT**
**Postfach 80 03 20**
**W-6230 Frankfurt am Main 80(DE)**

(72) Erfinder: **Dukat, Wolfgang, Dr.**

**Seilerbahnweg 2a**
**W-6240 Königstein/Ts.(DE)**
Erfinder: **Kutzner, Bernd**
**Johann-Strauss-Strasse 25**
**W-6233 Kelkheim/Ts.(DE)**
Erfinder: **Siegemund, Günter, Dr.**
**Frankfurter Strasse 21**
**W-6238 Hofheim/Ts.(DE)**
Erfinder: **Wanzke, Wolfgang, Dr.**
**Eichendorffstrasse 1**
**W-8906 Gersthofen(DE)**

(54) **Verfahren zur Trennung eines Gemischs, enthaltend Fluorwasserstoff und 1-Chlor-2,2,2-trifluorethan.**

(57) Die Erfindung betrifft ein Verfahren zur Trennung eines Gemischs, enthaltend Fluorwasserstoff und 1-Chlor-2,2,2-trifluorethan, bei dem man das Gemisch auf eine Temperatur von −80°C bis −20°C in einer Trennungszone abkühlt, wobei eine organische Phase mit einem HF-Gehalt von weniger als 10 mol-% als untere Phase und eine HF-Phase mit einem HF-Gehalt von mehr als 95 mol-% als obere Phase in der Trennungszone gebildet werden.

EP 0 542 290 A1

Die Erfindung betrifft ein Verfahren zur Trennung eines Gemischs, enthaltend Fluorwasserstoff (HF) und 1 − Chlor − 2,2,2 − trifluorethan (R 133a).

R 133a ist ein vielseitiges Zwischenprodukt bei der Herstellung von teilhalogenierten Fluorkohlenwas − serstoffen (H − FKW) bzw. Fluorchlorkohlenwasserstoffen (H − FCKW), die als Ersatzstoffe für vollhaloge − nierte FCKW auf verschiedenen Anwendungsgebieten dienen und diesen ökologisch überlegen sind. R 133a kann durch weitere Reaktionen sowohl in R 134a (1,1,1,2 − Tetrafluorethan) als auch in R 123 (1,1 − Dichlor − 2,2,2 − trifluorethan), R 124 (1 − Chlor − 1,2,2,2 − tetrafluorethan) und R 125 (Pentafluorethan) um − gewandelt werden. Ebenso ist es als Vorstufe zur Synthese des Inhalationsanästhetikums 1 − Brom − 1 − chlor − 2,2,2 − trifluorethan und zur Herstellung von Trifluoressigsäurederivaten einsetzbar.

Für einen technischen Prozeß zur Herstellung von aliphatischen Fluorkohlenwasserstoffen aus Chlor − kohlenwasserstoffen durch Umsetzung mit Fluorwasserstoff ist die Rückgewinnung von überschüssigem HF, bzw. dessen Rückführung in den Reaktionsablauf aus mehreren Gründen bedeutsam. Zum einen sprechen wirtschaftliche Überlegungen für eine möglichst vollständige Ausnutzung des wasserfreien Fluorwasserstoffs im Verfahren, da die Umwandlung in wäßrige Flußsäure oder Fluoridlösungen einen beträchtlichen Wert − verlust bedeutet. Zum anderen sollten fluoridhaltige Abwässer aus Gründen des Gewässerschutzes ver − mieden werden.

Es ist bereits bekannt, daß flüssiger Fluorwasserstoff mit vielen aliphatischen Fluorkohlenwasserstoffen nur begrenzt mischbar ist. Diese Tatsache ist auch technisch bereits ausgenutzt worden, indem man die heterogenen Mischungen in einem Phasenabscheider in eine HF − reichere und eine HF − ärmere Phase aufgetrennt hat, um die beiden Phasen anschließend getrennt weiterzuverarbeiten.

US − PS 2 450 415 beschreibt beispielsweise eine Phasentrennung zwischen Dichlordifluormethan (R 12) und HF. Die R 12 − Phase mit niedrigem HF − Gehalt wird anschließend wäßrig aufgearbeitet, während die HF − reiche Phase in die Reaktion zurückgeführt wird.

US − PS 2 478 362 beschreibt die Phasentrennung zwischen HF und den Fluorierungsprodukten von Tetrachlorethen in einem kontinuierlichen Herstellverfahren. Die HF − reiche Phase wird auch hier zum Reaktor zurückgeführt.

US − PS 3 947 558 beschreibt eine Folge von zwei Phasentrennungen, bei denen HF möglichst vollständig aus einem Fluorierungsprozeß für $C_1 − C_3$ − Fluorkohlenwasserstoffe zurückgewonnen wird. Zu − erst wird eine HF − arme organische Phase abgetrennt, die dann mit einem Monoglykol versetzt wird, in dem die fluororganischen Verbindungen praktisch unlöslich sind. Nach der zweiten Phasenscheidung befindet sich HF nahezu ausschließlich in der Glykolphase und kann daraus durch Destillation zurückge − wonnen werden.

EP − B − 98 341 beschreibt die Rückgewinnung von HF aus dessen Gemischen mit Pentafluorbutan und 1 − Chlor − 1,1 − difluorethan durch Phasentrennung und Destillation.

US − PS 4 911 792 (= EP − A − 0 353 970) beschreibt die Trennung von Mischungen aus HF, 2,2 − Dichlor − 1,1,1 − trifluorethan und/oder 2 − Chlor − 1,1,1,2 − tetrafluorethan durch Phasenabscheidung und anschließende Destillation beider Phasen.

Die katalytische Herstellung von R 133a aus Trichlorethen und HF wird in der EP − A − 0 407 961 beschrieben. Das bei dieser Reaktion erhaltene Produktgemisch enthält außer R 133a und überschüssigem HF noch Chlorwasserstoff, der durch Destillation abgetrennt werden kann. Eine Trennung von R 133a und HF ist jedoch durch einfache Destillation nicht möglich.

Gegenstand der vorliegenden Erfindung ist ein Verfahren zur Trennung eines Gemischs, enthaltend Fluorwasserstoff und 1 − Chlor − 2,2,2 − trifluorethan, dadurch gekennzeichnet, daß man das Gemisch auf eine Temperatur von − 80˚C bis − 20˚C in einer Trennungszone abkühlt, wobei eine organische Phase mit einem HF − Gehalt von weniger als 10 mol − % als untere Phase und eine HF − Phase mit einem HF − Gehalt von mehr als 95 mol − % als obere Phase in der Trennungszone gebildet werden.

Durch die gewählte tiefe Temperatur weisen die beiden Phasen eine Zusammensetzung auf, die eine vollständige Trennung von HF und R 133a durch anschließende Destillation der Phasen ermöglicht.

Zwar werden unter Normaldruck bereits bei 0˚C zwei Phasen gebildet, während ein entsprechendes Gemisch bei 20˚C noch homogen vorliegt. Die Zusammensetzung der beiden Phasen ist jedoch bei 0˚C noch nicht so weit von den Konzentrationen im eingebrachten Rohgemisch entfernt, daß mit einer getrennten Destillation beider Phasen bereits eine vollständige Trennung der Komponenten R 133a und HF möglich wäre.

Bei − 20˚C sind dagegen die Konzentrationsunterschiede in den Phasen unter Normaldruck bereits groß genug. Man erhält bei der Phasentrennung eine R 133a − Phase mit weniger als 10 mol − % HF und eine HF − Phase mit weniger als 5 mol − % R 133a. Bevorzugt wird die Phasentrennung bei − 40˚C bis − 80˚C durchgeführt: hier findet man in der R 133a − Phase nur noch weniger als 2 % HF und in der HF − Phase weniger als 1 % R 133a.

Der Druck beträgt im allgemeinen 0,1 bis 10 bar, vorzugsweise 1 − 5 bar.

Durch die erfindungsgemäße Phasentrennung bei tiefer Temperatur wird eine obere HF − Phase mit hohem HF − Gehalt und niedrigem R 133a − Gehalt erhalten, sowie eine untere organische Phase mit niedrigem HF − Gehalt und hohem R 133a − Gehalt.

Vorzugsweise wird die HF − Phase aus der Trennungszone entfernt, in eine Destillationskolonne über − führt und an deren Sumpf HF abgezogen, während man ein Azeotrop aus HF und 1 − Chlor − 2,2,2 − trifluorethan am Kopf der Kolonne gewinnt. Die Temperatur am Kolonnenkopf beträgt dabei im allgemeinen − 40 ˚ C bis + 80 ˚ C, vorzugsweise 0 ˚ C bis + 50 ˚ C. Der Druck in der Kolonne beträgt im allgemeinen 0,1 bar bis 10 bar, vorzugsweise 1 bar bis 5 bar (jeweils absolute Druckwerte). Das Azeotrop wird anschließend vorzugsweise in die Trennungszone zurückgeführt.

Alternativ kann die HF − Phase statt zur destillativen Trennung in HF und Azeotrop auch in den Reaktor zurückgeführt werden, in dem die Umsetzung von Trichlorethen mit HF zu R 133a stattfindet.

In einer besonders bevorzugten Ausführungsform wird zusätzlich zur HF − Phase auch die organische Phase destillativ aufgearbeitet, indem man sie aus der Trennungszone entfernt, in eine Destillationskolonne überführt und an deren Sumpf 1 − Chlor − 2,2,2 − trifluorethan abzieht, während man am Kopf der Kolonne ein Azeotrop aus HF und 1 − Chlor − 2,2,2 − trifluorethan gewinnt. Die Temperatur am Kopf dieser Kolonne beträgt dabei im allgemeinen − 50 ˚ C bis + 80 ˚ C, vorzugsweise 0 ˚ C bis + 50 ˚ C. Der Druck in der Kolonne beträgt im allgemeinen 0,1 bis 10 bar, vorzugsweise 1 bis 5 bar (jeweils absolute Druckwerte, wie auch alle anderen Druckangaben in der vorliegenden Beschreibung). Das Azeotrop wird vorzugsweise wieder in die Trennungszone zurückgeführt.

Indem man das als Kopfprodukt der beiden Destillationen erhaltene Azeotrop aus HF und R 133a immer wieder in die Trennungszone zurückführt und dort mit frischem Ausgangsgemisch (enthaltend HF und R 133a) vereinigt, wird im kontinuierlichen Betrieb eine vollständige Trennung von HF und R 133a erreicht.

Zum Einsatz des gemäß EP − A − 0 407 961 (siehe oben) erhaltenen Produktgemischs wird dieses zunächst destillativ von HCl befreit. Das dabei erhaltene Gemisch enthält im allgemeinen noch kleine Mengen höhersiedende Verbindungen, wie nicht umgesetztes Trichlorethen und 1,2 − Dichlor − 1,1 − difluo − rethan (R 132b), die den Ablauf des erfindungsgemäßen Verfahrens nicht beeinträchtigen. Tiefsiedende Verunreinigungen, z.B. Pentafluorethan (R 125) werden zusammen mit HCl bereits vorher abdestilliert, so daß in der Trennungszone nur noch ein Gemisch aus R 133a und HF mit einem kleinen Anteil (<3 %) an den genannten höhersiedenden Verbindungen vorliegt.

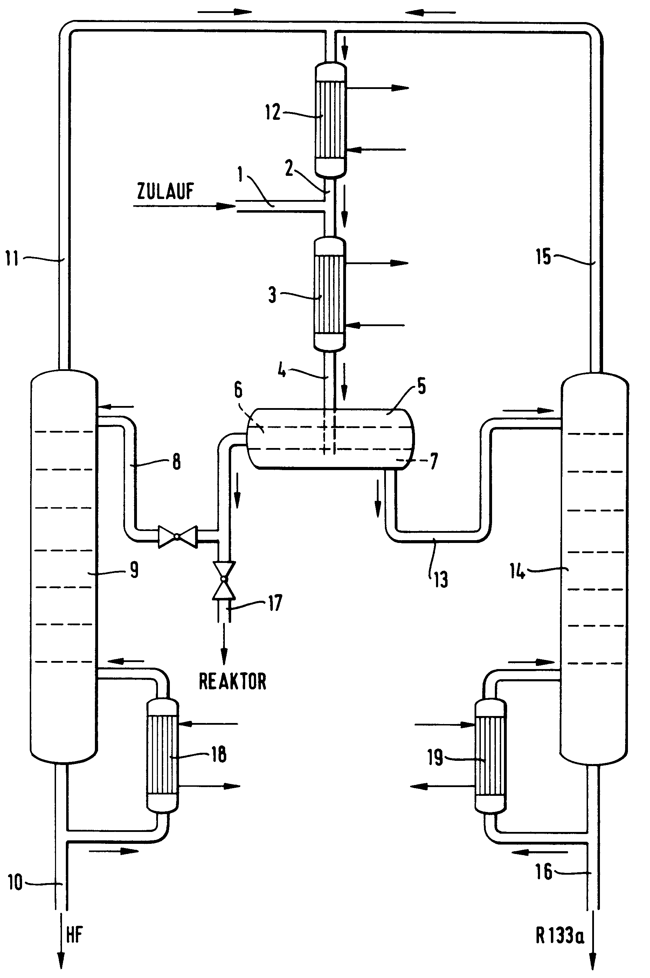

Figur 1 ist eine schematische Darstellung einer bevorzugten Ausführungsform der Erfindung.

Über Leitung (1) und (2) strömt ein Produktgemisch, das im wesentlichen aus R 133a und HF (mit kleinen Mengen an nicht umgesetztem Trichlorethen und geringfügigen Verunreinigungen, wie 1,2 − Dichlor − 1,1 − difluorethan) besteht, über den Kühler (3) und Leitung (4) zum Phasenabscheider (Trennungszone) (5). Im Phasenabscheider (5) bildet sich eine obere HF − reiche Phase (6) und eine untere R 133a − reiche Phase (7). Die HF − Phase (6) wird dem Phasenabscheider (5) entzogen und über Leitung (8) in die Destillationskolonne (9) eingespeist. Am Sumpf der Kolonne (9) wird reiner Fluorwasserstoff über Leitung (10) zurückgewonnen. Das am Kolonnenkopf anfallende Azeotrop aus HF und R 133a wird über Leitung (11) in den Kondensator (12) geleitet und dort kondensiert und dann über Leitung (2) und Kühler (3) dem Phasenabscheider (5) wieder zugeführt.

Um das R 133a aus der unteren organischen Phase (7) zu gewinnen, wird diese aus dem Phasenab − scheider (5) abgezogen und über Leitung (13) einer zweiten Destillationskolonne (14) zugeführt. Am Kolonnenkopf wird über Leitung (15) ein Azeotrop, bestehend aus HF und R 133a, abgezogen, im Kondensator (12) verflüssigt und über den Kühler (3) dem Phasenabscheider (5) zugeführt. R 133a wird am Sumpf von Kolonne (14) über Leitung (16) abgezogen. Über Leitung (17) kann die obere Phase (6) aus dem Phasenabscheider (5) auch ganz oder teilweise in den Reaktor zurückgeführt werden, was aber nicht bevorzugt ist. Mit den beiden Umlaufverdampfern (18) und (19) werden die Kolonnen (9) und (14) betrieben.

Die folgenden Beispiele erläutern die Erfindung.

Beispiele

Vergleichsbeispiel 1

10 g HF (0,5 mol) und 56,2 g R 133a (0,47 mol) wurden in ein gasdichtes Gefäß aus transparentem Fluorkunststoff (Polychlortrifluorethylen PCTFE) gefüllt. Nach Temperierung des Gefäßes auf 20 ˚ C war keine Phasentrennung erkennbar. Auch nach 24 h war die Mischung homogen. Analysenproben aus dem unteren und dem oberen Teil der Mischung enthielten die gleiche molare Konzentration an HF bzw. R 133a.

Vergleichsbeispiel 2

504 g HF (25,2 mol) und 496 g R 133a (4,2 mol) wurden in einem Stahlzylinder vermischt und bei 20°C temperiert. Nach 5 h wurden aus dem unteren und dem oberen Teil der Mischung jeweils 50 g entnommen und analysiert. Die gemessenen HF − bzw. R 133a − Konzentrationen stimmten mit dem eingewogenen Verhältnis überein, so daß bei einem Molverhältnis HF/R 133a von 6:1 ebenfalls keine Phasentrennung erfolgte.

Vergleichsbeispiel 3

Die Mischung aus Vergleichsbeispiel 1 wurde auf eine Temperatur von 0°C abgekühlt. Innerhalb weniger Minuten wurde die Bildung von 2 Phasen beobachtet.

Aus beiden Phasen wurden Proben entnommen und die Konzentrationen an HF bzw. R 133a bestimmt.

| Analyse: | | |
|---|---|---|
| | Obere Phase | Untere Phase |
| HF | 58 mol − % | 46 mol − % |
| R 133a | 42 mol − % | 54 mol − % |

Hier erfolgte zwar eine Phasentrennung, aber die beiden Phasen haben noch fast dieselbe Zusammensetzung.

Beispiel 1

Die Mischung aus Vergleichsbeispiel 1 wurde auf eine Temperatur von − 40°C abgekühlt. Anschließend wurden die beiden Phasen erneut analysiert.

| | Obere Phase | Untere Phase |
|---|---|---|
| HF | 99 mol − % | 2 mol − % |
| R 133a | 1 mol − % | 98 mol − % |

Beispiel 2

Für eine kontinuierlich arbeitende Trennapparatur nach Figur 1 wurde berechnet, welche Mengen an R 133a und HF an den Stellen (8), (10), (11), (13), (15) und (16) der Trennapparatur vorliegen würden, wenn bestimmte angenommene Mengen an R 133a und HF am Zulauf (1) eingespeist würden. Für die Trennleistung der Destillationskolonnen (9) und (14) wurden je 10 theoretische Böden angenommen. Die in (1) angenommenen Mengen und die an den anderen Stellen berechneten Mengen werden in der folgenden Tabelle angegeben; diese enthält außerdem die an allen genannten Stellen angenommenen und bei der Berechnung benutzten Temperatur − und Druckwerte. Bei der Berechnung wurden außerdem gemessene und berechnete thermodynamische Daten von R 133a und HF und deren Mischungen verwendet, insbesondere das Ergebnis von Beispiel 1, betreffend die Phasentrennung bei − 40°C.

| Menge | (1) | (8) | (10) | (11) | (13) | (15) | (16) |
|---|---|---|---|---|---|---|---|
| F 133a (kmol/h) | 48,70 | 1,635 | - | 1,639 | 51,176 | 2,472 | 48,70 |
| HF (kmol/h) | 51,30 | 53,235 | 51,30 | 1,924 | 1,206 | 1,217 | - |
| Temperatur (°C) | 20 | -40 | 52 | 33,8 | -40 | 27,4 | 36,2 |
| Druck (bar) | 3 | 3 | 3 | 3 | 3 | 3 | 3 |

(1) : Zulauf des Produktgemischs

(8) : HF-Phase aus dem Phasenabscheider (5)

(10): Sumpfprodukt von Kolonne (9)

(11): Kopfprodukt von Kolonne (9)

(13): F 133a-Phase aus dem Phasenabscheider (5)

(15): Kopfprodukt von Kolonne (14)

(16): Sumpfprodukt von Kolonne (14)

**Patentansprüche**

1. Verfahren zur Trennung eines Gemischs, enthaltend Fluorwasserstoff und 1 – Chlor – 2,2,2 – trifluoret – han, dadurch gekennzeichnet, daß man das Gemisch auf eine Temperatur von – 80 ˚ C bis – 20 ˚ C in einer Trennungszone abkühlt, wobei eine organische Phase mit einem HF – Gehalt von weniger als 10 mol – % als unterePhase und eine HF – Phase mit einem HF – Gehalt von mehr als 95 mol – % als obere Phase in der Trennungszone gebildet werden.

2. Verfahren nach Anspruch 1, dadurch gekennzeichnet, daß man das Gemisch auf −40°C bis −80°C abkühlt.

3. Verfahren nach Anspruch 1 oder 2, dadurch gekennzeichnet, daß man die HF – Phase aus der Trennungszone entfernt, in eine Destillationskolonne überführt und an deren Sumpf HF abzieht, während man ein Azeotrop aus HF und 1 – Chlor – 2,2,2 – trifluor – ethan am Kopf der Kolonne gewinnt.

4. Verfahren nach Anspruch 3, dadurch gekennzeichnet, daß die Temperatur am Kolonnenkopf −40°C bis +80°C beträgt.

5. Verfahren nach Anspruch 3 oder 4, dadurch gekennzeichnet, daß man das Azeotrop in die Trennungs – zone zurückführt.

6. Verfahren nach einem der Ansprüche 1 bis 5, dadurch gekennzeichnet, daß man die organische Phase aus der Trennungszone entfernt, in eine Destillationskolonne überführt und an deren Sumpf 1 – Chlor – 2,2,2 – trifluorethanabzieht, während man am Kopf der Kolonne ein Azeotrop aus HF und 1 – Chlor – 2,2,2 – trifluorethan gewinnt.

7. Verfahren nach Anspruch 6, dadurch gekennzeichnet, daß die Temperatur am Kolonnenkopf −50°C bis +80°C beträgt.

8. Verfahren nach Anspruch 6 oder 7, dadurch gekennzeichnet, daß man das Azeotrop in die Trennungs – zone zurückführt.

ZULAUF

REAKTOR

HF

R 133a

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| P,X | EP-A-0 509 885 (ELF ATOCHEM S. A.) <br> * das ganze Dokument * <br> --- | 1-8 | C07C17/38 <br> C07C19/08 <br> C01B7/19 |
| P,X | EP-A-0 509 449 (DAIKIN INDUSTRIES) <br> * Ansprüche 8-9; Beispiel 7 * <br> --- | 1,3-8 | |
| D,X | EP-A-0 353 970 (E. I. DU PONT DE NEMOURS) <br> * Ansprüche; Beispiele * <br><br> ----- | 1-8 | |
| | | | **RECHERCHIERTE SACHGEBIETE (Int. Cl.5 )** <br><br> C07C <br> C01B |

Der vorliegende Recherchenbericht wurde für alle Patentansprüche erstellt

| Recherchenort | Abschlußdatum der Recherche | Prüfer |
|---|---|---|
| DEN HAAG | 23 FEBRUAR 1993 | ZERVAS B. |